# EUROPEAN PATENT SPECIFICATION

(11) **EP 2 818 861 B1**
(45) Date of publication and mention of the grant of the patent: **25.10.2017**
(21) Application number: 13173817.1
(22) Date of filing: 26.06.2013
(51) Int. Cl.: G01N 33/00, G01N 33/02, G01N 33/50, G01N 33/68, G01N 33/569, G01N 30/72

(54) **Method for predicting the sugar content in a full-grown root vegetable**
Verfahren zur Vorhersage des Zuckergehalts in einem voll ausgewachsenen Wurzelgemüse
Procédé de prédiction de la teneur en sucre d'un légumes-racine arrivé à maturité

(43) Date of publication of application: 31.12.2014
(73) Proprietor: Metabolomic Discoveries GmbH, 14476 Potsdam (DE)
(72) Inventor: Schauer, Nicolas, 14476 Potsdam (DE); Trenkamp, Sandra, 40215 Düsseldorf (DE); Hische, Manuela, 14467 Potsdam (DE)
(74) Representative: Geling, Andrea

(56) References cited:
- WO-A2-2005/017646
- US-A1- 2013 139 277
- UTE ROESSNER ET AL: "Simultaneous analysis of metabolites in potato tuber by gas chromatography-mass spectrometry", THE PLANT JOURNAL, vol. 23, no. 1, 1 July 2000 (2000-07-01), pages 131-142, XP055079539, ISSN: 0960-7412, DOI: 10.1046/j.1365-313x.2000.00774.x
- D. GLASSOP ET AL: "Changes in the Sugarcane Metabolome with Stem Development. Are They Related to Sucrose Accumulation?", PLANT AND CELL PHYSIOLOGY, vol. 48, no. 4, 16 February 2007 (2007-02-16), pages 573-584, XP055079413, ISSN: 0032-0781, DOI: 10.1093/pcp/pcm027
- J.-L. WOLFENDER: "Plant metabolomics: From holistic data to relevant biomarkers", CURRENT MEDICINAL CHEMISTRY, vol. 20, no. 8, 1 January 2013 (2013-01-01), pages 1056-1090, XP55079422, ISSN: 0929-8673
- ROGGO Y ET AL: "Sucrose content determination of sugar beets by near infrared reflectance spectroscopy. Comparison of calibration methods and calibration transfer", JOURNAL OF NEAR INFRARED SPECTROSCOPY, NIR PULBICATIONS, CHICHESTER, GB, vol. 10, 1 January 2002 (2002-01-01), pages 137-150, XP002590800, ISSN: 0967-0335

## Description

The present invention relates to a method for predicting the sugar content in a full-grown sugar beet. The present invention further relates to the use of metabolites for predicting the sugar content in a full-grown sugar beet.

### BACKGROUND OF THE INVENTION

Plant breeding is the art and science of changing the genetics of plants in order to produce desired traits. In particular, the aim of plant breeding is to improve the quality and performance of plants. Historically, plant breeding started with sedentary agriculture and particularly the domestication of the first agricultural plants. Initially early human farmers simply selected food plants with particular desirable characteristics, and used these as a seed source for subsequent generations, resulting in an accumulation of characteristics over time. In time, however, experiments began with deliberate crossings, the science and understanding of which was greatly enhanced centuries later by the work of Mendel. Mendel's work ultimately led to the new science of genetics. Modern plant breeding involves applied genetics, but it also covers molecular biology, cytology, physiology, pathology, entomology, chemistry, and statistics. The problem of plant breeding is, however, that it is complex, time and cost consuming.

One biological interesting trait is the sugar content in sugar containing or sugar storing plants. In particular, the cultivation of sugar containing or sugar storing plants having a high sugar content is of great interest for the agronomical industry. As mentioned above, breeding of plants having specific characteristics like a high sugar content is complex. In addition, no time- and cost-effective method and no reliable and highly predictive molecular marker is presently available allowing the prediction of the sugar content in sugar containing or sugar storing plants at an early growth stage and the subsequent selection and accumulation of sugar containing or sugar storing plants predicted to have a high sugar content when full-grown.

There are several prior art documents known. ROESSNER U. et al. (THE PLANT JOURNAL, 2000, Vol. 23, No. 1, pages 131-142), for example, describe the simultaneous analysis of metabolites in potato tuber by gas chromatography-mass spectrometry.

WO 2005/017646 A2 provides a method, system and software to screen for, identify and validate biomarkers that are predictive of a biological state, such as a cell state and/or patient status.

GLASSOP D. et al. (PLANT AND CELL PHYSIOLOGY, 2007, Vol. 48, No. 4, pages 573-584) refer to changes in the sugarcane metabolome with stem development and raises the question whether they are related to sucrose accumulation.

WOLFENDER J.-L. et al. (CURRENT MEDICINAL CHEMISTRY, 2013, Vol. 20, No. 8, pages 1056-1090) describe plant metabolomics: from holistic data to relevant biomarkers.

US 2013/0139277 A1 relates to agricultural business methods that may be sued in conjunction with a novel technology that can predict the yield of a given plant.

ROGGO Y. et al. (JOURNAL OF NEAR INFRARED SPECTROSCOPY, NIR PULBICATIONS, CHICHESTER, GB, 2002, Vol. 10, pages 137-150) relate to sucrose content determination of sugar beets by near infrared reflectance spectroscopy. Comparison of calibration methods and calibration transfer were made.

The present inventors surprisingly found that the sugar content in sugar containing or sugar storing plants is predictable by using metabolite compositions. In other words, they surprisingly found that metabolite compositions are able to support the discovery process of sugar containing or sugar storing plants having a high sugar content. Thereby, the metabolites function as early selection biomarkers allowing a significant acceleration of the breeding, selection and accumulation processes. In particular, the present inventors surprisingly found a high correlation between the sugar content in full-grown root vegetables, e.g. sugar beets, and the presence of specific metabolite compositions which can already be detected in root vegetable seedlings, e.g. sugar beet seedlings. This surprising finding allows predicting the future sugar content of root vegetables, e.g. sugar beets, solely on the basis of specific metabolite compositions, particularly in leaf, stem, or other plant tissue, and at an early stage of root vegetable development, e.g. sugar beet development. Surprisingly, the metabolites having the above mentioned predictive value are not involved in the sugar anabolism and/or catabolism.

### SUMMARY OF THE INVENTION

In a first aspect, the present invention relates to a method for predicting the sugar content in a full-grown sugar beet comprising the steps of:
(i) determining the level of each one of at least two metabolites selected from the group consisting of Var878, Var62, Var37, and Var19 in a sample obtained from a sugar beet seedling, wherein
   Var878 is 2-oxo-glutaric acid, has an accurate mass of 146.0218 Da ± 3 ppm, and a retention time of 0.399 min ± 0.25 min in liquid chromatography (LC),
   Var62 is phosphoric acid, has a retention index of 1262.34 ± 2, and shows fragments having a nominal mass of 314, 299, 211, 283, and 225 Da in mass spectrometry (MS),
   Var37 is oxamide, has a retention index of 1328.63 ± 2, and shows fragments having a nominal mass of 102, 189, 117, 217, and 131 Da in mass spectrometry (MS), Var19 is fumaric acid, has a retention index of 1347.49 ± 2, and shows fragments having a nominal mass of 245, 115, 217, 143, and 133 Da in mass spectrometry (MS); and
(ii) comparing the level of each metabolite to a reference, wherein said comparison allows predicting the sugar content in the full-grown sugar beet, and/or
   applying an algorithm or a mathematical function to the levels of the metabolites, wherein said application allows predicting the sugar content in the full-grown sugar beet.

In a second aspect, the present invention relates to the use of at least two metabolites selected from the group consisting of Var878, Var62, Var37, and Var19 for predicting the sugar content in a full-grown sugar beet, wherein Var878, Var62, Var37, and Var19 are defined as in the method according ot the first aspect.

### BRIEF DESCRIPTION OF THE FIGURES

The following Figures are merely illustrative of the present invention and should not be construed to limit the scope of the invention as indicated by the appended claims in any way.
- **Figure 1:**: Relative sugar content in % for each investigated genotype. Dark bars indicate the four genotypes that were meant to form the reference (100%, horizontal line).
- **Figure 2:**: List of metabolites selected to be relevant for prediction of the relative sugar content. Metabolites are grouped according to their increasing importance as described in **Table 2.** Each metabolite is defined by its chromatographic and mass spectrometric features.
- **Figure 3:**: List of metabolites selected to be relevant for prediction of the category of relative sugar content. Metabolites are grouped according to their increasing importance as described in **Table 5.** Each metabolite is defined by its chromatographic and mass spectrometric features.

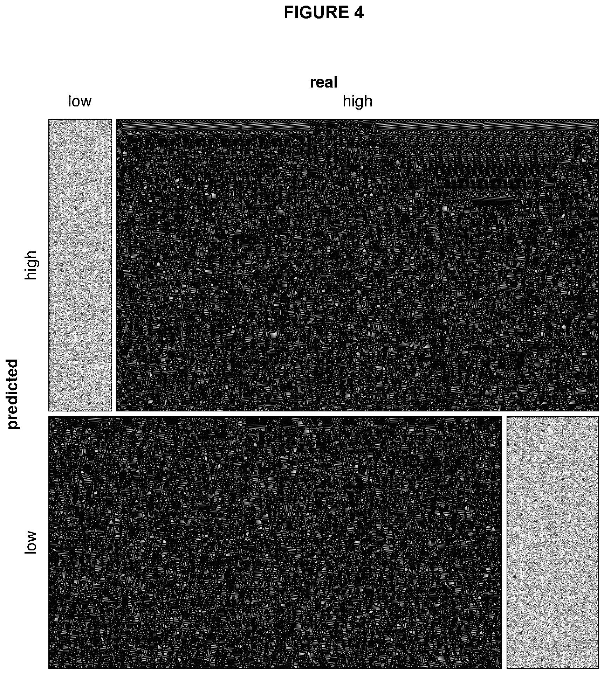

- **Figure 4:**: Deviation of predicted (rows) and real (columns) cases of low and high relative sugar content. Dark tiles represent correctly classified values, whereas light tiles represent incorrectly classified values. The prediction was made using the more complex classification rules described in **Table 7.**

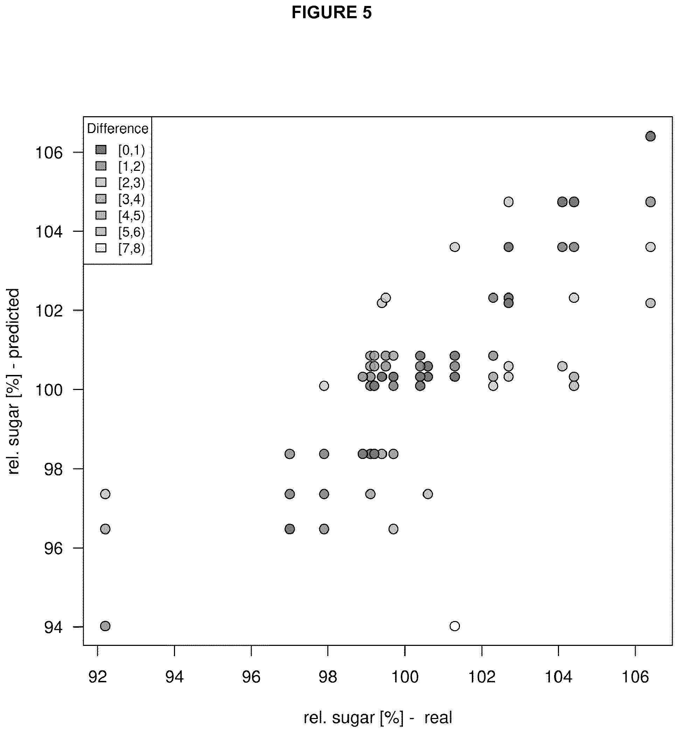

- **Figure 5:**: Predicted vs. real relative sugar content [%] after applying the regression model described in **Table 4.** Grey shades represent the difference between real and predicted values. The correlation between real and predicted values was 0.86. The mean squared error (MSE) was 2.64.
- **Figure 6:**: List of metabolites with their respective accurate mass in Da and the conversion of 3 ppm, 2.5 ppm and 2 ppm into Da.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, definitions will be provided which apply to all aspects of the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

As used in this specification and the appended claims, the singular forms "a", "an", and "the" include plural referents, unless the content clearly dictates otherwise.

The term "predicting the sugar content in a full-grown root vegetable", as used herein, means that the future sugar content in a full-grown root vegetable is anticipated. This anticipation is based on the metabolite profile in a root vegetable seedling which allows the prediction of the sugar content that will be exhibited at a full-grown root vegetable stage.

In this respect, it should be noted that with the predicted sugar content, the sugar content in the root of the full-grown root vegetable is meant.

The term "root vegetable", as used herein, refers to a plant root that is used as edible vegetable or processable raw material. Here, the term "root" means any underground part of a plant. A root vegetable is generally a storage organ, enlarged to store energy in form of carbohydrates like sugar and starch. A root vegetable particularly contains sugar or stores sugar in its root. Thus, the term "root vegetable" encompasses sugar containing or sugar storing plants. The root vegetable may be a beet (e.g. a sugar beet, forage beet, beetroot, or turnip), carrot, parsnip, crummock, or potato (e.g. a sweet potato or table potato). According to the present invention, the root vegetable is a sugar beet.

The term "full-grown root vegetable", as used herein, refers to a root vegetable that has reached its full adult size and stopped growing. The term "full-grown root vegetable", as used herein, also refers to a root vegetable that is mature or ripe. Usually, a root vegetable is harvested and consumed, when it has reached its full adult size and stopped growing, or when it is mature or ripe. The skilled person knows about the different cultivation times of root vegetables. For example, sugar beets are planted in spring and harvested in autumn in most temperature climates. A full-grown sugar beet weights, for example, between 700 and 800 g.

The term "root vegetable seedling", as used herein, refers to a root vegetable that has not yet reached its full adult size and is still growing. The term "root vegetable seedling", as used herein, also refers to a root vegetable seedling that is not mature or ripe. The term "root vegetable seedling", as used herein, further means a young root vegetable that has been grown from a seed or seed root vegetable e.g. seed potato. The root vegetable seedling, e.g. beet seedling such as sugar beet seedling, may be a seedling at the 2- to 8-leaf stage, e.g. at the 2-leaf stage, 3-leaf stage, 4-leaf stage, 5-leaf stage, 6-leaf stage, 7-leaf stage, or 8-leaf stage.

The term "sugar content", as used herein, refers to the sugar concentration that is present in the root of the full-grown root vegetable. Sugar is formed through a process of photosynthesis in that parts of the root vegetable that are exposed to sunlight, e.g. in the leaves and stem, during root vegetable development. The sugar is then stored in the root. The root of a sugar beet contains, in addition to sugar, for example, water and minerals.

The term "sample obtained from a root vegetable seedling", as used herein, refers to any sample of a root vegetable seedling allowing the prediction of the sugar content in a full-grown root vegetable. As mentioned above, sugar is formed in that parts of the root vegetable that are exposed to sunlight, e.g. in the leaves and stem, during root vegetable development. Thus, the term "sample obtained from a root vegetable seedling", as used herein, also refers to any sample of a root vegetable seedling that is exposed to sunlight. The sample obtained from a root vegetable seedling may be a leaf or a stem.

The term "reference", as used herein, refers to any reference which allows predicting the sugar content in a full-grown root vegetable.

In its simplest form, said reference may be a threshold (metabolite) level representative for a specific sugar content. The measured (metabolite) level may be identically equal to, below, or above the threshold (metabolite) level representative for a specific sugar content. If the measured (metabolite) level is, for example, identically equal to or above said threshold (metabolite) level, the root vegetable is predicted to have at least the specific sugar content when full-grown. If the measured level is, for example, below said threshold (metabolite) level, the root vegetable is predicted to have a lower sugar content as the specific sugar content when full-grown.

Said reference may also be a threshold (metabolite) level representative for a specific sugar content category, e.g. a high, middle, or low sugar content category. The measured (metabolite) level may be identically equal to, below, or above the threshold (metabolite) level representative for a specific sugar content category. If the measured (metabolite) level is, for example, identically equal to the threshold (metabolite) level representative for a high sugar content category, the root vegetable is predicted to have a high sugar content when full-grown. Further, if the measured level is, for example, identically equal to the threshold (metabolite) level representative for a middle sugar content category, the root vegetable is predicted to have a middle sugar content when full-grown. Furthermore, if the measured level is, for example, identically equal to the threshold (metabolite) level representative for a low sugar content category, the root vegetable is predicted to have a low sugar content when full-grown.

Said reference may further be a (metabolite) level range representative for a specific sugar content category, e.g. a high, middle, or low sugar content category. The measured (metabolite) level may fall within said range, e.g. within the range representative for a high, middle, or low sugar content category. If the measured (metabolite) level, for example, falls within the (metabolite) level range representative for a high sugar content category, the root vegetable is predicted to have a high sugar content when full-grown, if the measured (metabolite) level, for example, falls within the (metabolite) level range representative for a middle sugar content category, the root vegetable is predicted to have a middle sugar content when full-grown, or if the measured (metabolite) level, for example, falls within the (metabolite) level range representative for a low sugar content category, the root vegetable is predicted to have a low sugar content when full-grown.

The term "algorithm or mathematical function", as used herein, refers to any algorithm or mathematical function which allows predicting the sugar content in a full-grown root vegetable.

The term "mass spectrometry (MS)", as used herein, refers to the science of displaying the *spectra* (singular *spectrum*) of the masses of the molecules comprising a sample of material. It is used for determining the elemental composition of a sample, the masses of particles and of molecules, and for elucidating the chemical structures of molecules, such as metabolites. Mass spectrometry works by ionizing chemical compounds to generate charged molecules or molecule fragments and measuring their mass-to-charge (m/z) ratios.

The mass spectrometry may be an electrospray ionization mass spectrometry (ESI-MS). Electrospray ionization is a soft ionization technique. It can, thus, be used to determine the molecular weights of biological molecules such as metabolites. Soft ionization is a useful technique when considering biological molecules such as metabolites, because this process does not fragment the biological molecules such as metabolites into smaller charged particles, rather it turns the biological molecules being ionized into small droplets. These droplets will then be further desolvated into even smaller droplets, which creates molecules with attached protons. These protonated and desolvated molecular ions will then be passed through the mass analyzer to the detector, and the mass of the sample can be determined. Coupled with a high performance liquid chromatograph (HPLC) for molecular fractionation prior to mass spectrometric analysis, HPLC/ESI-MS is a very powerful technique capable of analyzing biological molecules such as metabolites of various polarities in a complex sample. Electrospray ionization mass spectrometry (ESI-MS) can also be coupled with gas chromatography (GC/ESI-MS).

The mass spectrometry may be an electron ionization mass spectrometry (EI-MS) (also referred to as electron impact ionization mass spectrometry). Electron ionization is the oldest and best-characterized of all the ionization methods. A beam of electrons passes through the gas-phase sample. An electron that collides with a neutral analyte molecule can knock off another electron, resulting in a positively charged ion. The ionization process can either produce a molecular ion which will have the same molecular weight and elemental composition of the starting analyte, or it can produce a fragment ion which corresponds to a smaller piece of the analyte molecule. The ionization potential is the electron energy that will produce a molecular ion. The appearance potential for a given fragment ion is the electron energy that will produce that fragment ion. Most mass spectrometers use electrons with an energy of 70 electron volts (eV) for El. Decreasing the electron energy can reduce fragmentation, but it also reduces the number of ions formed.

The mass spectrometry may further be a matrix-assisted laser desorption/ionization mass spectrometry or MALDI. In MALDI, a laser is used as an ionization source to generate gas phase ions from a sample on a surface and to detect the gas phase ions with a mass spectrometer. The analyte is typically mixed with a matrix material that, upon drying, co-crystallizes with the analyte. The matrix material absorbs energy from the energy source which otherwise would fragment the labile analytes.

The mass spectrometry may also be a surface-enhanced laser desorption/ionization mass spectrometry or SELDI. In SELDI, the surface on which the analyte is applied plays an active role in the analyte capture and/or desorption.

The term "tandem mass spectrometry (MS/MS)", as used herein, refers to multiple rounds of mass spectrometry, usually separated by some form of molecule fragmentation. For example, one mass analyzer can isolate one metabolite from many entering a mass spectrometer. A second mass analyzer then stabilizes the metabolite ions while they collide with a gas, causing them to fragment by collision-induced dissociation (CID). A third mass analyzer then sorts the fragments produced from the peptides. Tandem MS can also be done in a single mass analyzer over time, as in a quadrupole ion trap. There are various methods for fragmenting metabolites for tandem MS, including collision-induced dissociation (CID), electron capture dissociation (ECD), electron transfer dissociation (ETD), infrared multiphoton dissociation (IRMPD), blackbody infrared radiative dissociation (BIRD), electron-detachment dissociation (EDD) and surface-induced dissociation (SID).

In the context of the present invention, the term "apparent molecular mass" refers to the molecular mass (in Daltons)-to-charge value, m/z, of the detected ions. How the apparent molecular mass is derived is dependent upon the type of mass spectrometer used. With a time-of-flight mass spectrometer, the apparent molecular mass is a function of the time from ionization to detection.

In the context of the present invention, the term "signal" refers to any response generated by a molecule such as metabolite under investigation. For example, the term signal refers to the response generated by the metabolite hitting the detector of a mass spectrometer. The signal intensity correlates with the amount or concentration of the metabolite. The signal may be defined by two values: an apparent molecular mass value and an intensity value generated as described. The mass value is an elemental characteristic of the metabolite, whereas the intensity value accords to a certain amount or concentration of the metabolite with the corresponding apparent molecular mass value. Thus, the "signal" always refers to the properties of the metabolite.

The term "(measured) accurate mass", as used herein, refers to an experimentally determined mass that allows the elemental composition to be determined. The physical unit of the "(measured) accurate mass" is "Da" or "u".

The term "nominal mass", as used herein, refers to the mass of an ion or molecule that is calculated using the integer mass (ignoring the mass defect) of the most abundant isotope of each element. This is the equivalent to summing the mass numbers of all constituent atoms. For example H = 1, C = 12, O = 16, etc. The nominal mass of H₂O is 18, for example. The physical unit of the nominal mass" is "Da" or "u".

In the context of the present invention, the interval between the injection of a metabolite in a gas or liquid chromatograph and the detection of said metabolite is meant "retention time". Because retention times may vary between different metabolites, they can be used for metabolite characterization purposes.

The term "retention index (also designated as Kovats index)", as used herein, refers to an index used in gas chromatography to convert retention times into system-independent constants [1] [2]. The index is named after the Hungarian-born Swiss chemist Ervin Kováts, who outlined this concept during the 1950s while performing research into the composition of the essential oils. The retention index of a certain metabolite is its retention time normalized to the retention times of adjacently eluting n-alkanes. The retention index allows the comparison of values measured by different analytical laboratories under varying conditions.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

As mentioned above, the cultivation of sugar containing or sugar storing plants having a high sugar content is of great interest for the agronomical industry. The present inventors surprisingly found a high correlation between the sugar content in full-grown root vegetables, e.g. sugar beets, and the presence of a specific metabolite profile which can already be detected in root vegetable seedlings, e.g. sugar beet seedlings. This surprising finding allows predicting the future sugar content of root vegetables, e.g. sugar beets, solely on the basis of a specific metabolite profile and at an early stage of root vegetable development. Surprisingly, the metabolites having the above mentioned predictive value are not involved in the sugar anabolism and/or catabolism.

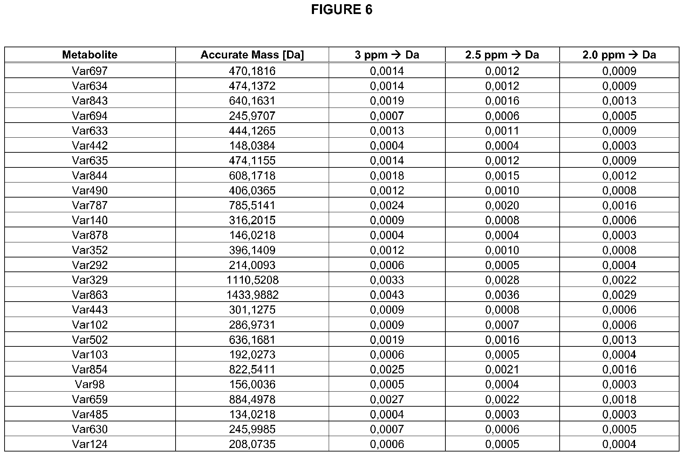

Thus, in a first aspect, the present invention relates to a method for predicting the sugar content in a full-grown sugar beet comprising the steps of:
(i) determining the level of each one of at least two metabolites selected from the group consisting of Var878, Var62, Var37, and Var19 in a sample obtained from a sugar beet seedling, wherein
   Var878 is 2-oxo-glutaric acid, has an accurate mass of 146.0218 Da ± 3 ppm, preferably ± 2.5 ppm, more preferably ± 2.0 ppm, and a retention time of 0.399 min ± 0.25 min in liquid chromatography (LC),Var62 is phosphoric acid, has a retention index of 1262.34 ± 2, and shows fragments having a nominal mass of 314, 299, 211, 283, and 225 Da in mass spectrometry (MS),
   Var37 is oxamide, has a retention index of 1328.63 ± 2, and shows fragments having a nominal mass of 102, 189, 117, 217, and 131 Da in mass spectrometry (MS), Var19 is fumaric acid, has a retention index of 1347.49 ± 2, and shows fragments having a nominal mass of 245, 115, 217, 143, and 133 Da in mass spectrometry (MS); and
(ii) comparing the level of each metabolite to a reference, wherein said comparison allows predicting the sugar content in the full-grown sugar beet, and/or
   applying an algorithm or a mathematical function to the levels of the (at least two) metabolites, wherein said application allows predicting the sugar content in the full-grown sugar beet.
   As to the conversion of parts per million (ppm) into Da, it is referred to **Figure 6****.**

As mentioned above, the level of each metabolite is compared to a reference. This is to be understood that each metabolite has a corresponding reference. In other words, different metabolites use/employ different references.

As mentioned above, the metabolite Var878 is characterized by its retention time in liquid chromatography (LC). It is preferred that the liquid chromatography (LC) is a reverse phase liquid chromatography (RP-LC). Preferably, the reverse phase liquid chromatography is performed with the solvents water and methanol with formic acid, e.g. with 0.1% formic acid, and/or a silica-based stationary phase. For example, the reverse phase liquid chromatography is conducted under conditions under which the concentration of water decreases from 100% to 0%, and the concentration of methanol with formic acid, e.g. with 0.1 % formic acid, increases from 0% to 100% (e.g. within 8 minutes at a flow rate of 0.4 ml/min). It is further preferred that the (reverse phase) liquid chromatography column used in (reverse phase) liquid chromatography (i) has a length of at least 10 mm, preferably of at least 50 mm, e.g. of 50 mm, (ii) has a diameter of at least 1.5 mm, preferably of at least 1.9 mm, e.g. of 1.9 mm, and/or (iii) has a film thickness of at least 1.5 µm, preferably of at least 1.8 µm, e.g. of 1.8 µm. It is, alternatively or additionally, further preferred that (i) the flow rate during (reverse phase) liquid chromatography is at least 0.2 ml/min, preferably is at least 0.4 ml/min, e.g. is 0.4 ml/min, and/or (ii) the (reverse phase) liquid chromatography is performed at a temperature of at least 20°C, preferably of at least 30°C, e.g. of 30°C. It is particularly preferred that the silica-based stationary phase is an octadecyl carbon chain (C18)-bonded silica-based stationary phase. For example, as liquid chromatography column, the Zorbax SB-Aq Rapid Resolution HD 2.1 x 50 mm 1.8 Micron reverse phase C18 column of Agilent Technologies is used. For example, as liquid chromatography system, the 1290 high pressure liquid chromatography (HPLC) system of Agilent Technologies is used (see experimental section).

As mentioned above, the metabolites Var62, Var37, and Var19 are characterized by their fragments detected in mass spectrometry (MS). Fragmentation is a type of chemical dissociation that takes place of said metabolites during mass spectrometry. When the vaporised sample comprising said metabolites passes into the ionisation chamber of a mass spectrometer, it is bombarded by a stream of electrons. These electrons have a high enough energy to knock an electron off said molecules to form a positive ion. This ion is called the molecular ion. The molecular ions are energetically unstable and some of them break up into smaller pieces, the above mentioned fragments. Said fragments are characteristic for the above mentioned metabolites. It is preferred that the mass spectrometry (MS) is an electron impact ionization mass spectrometry (EI-MS). Preferably, the mass spectrometry, such as electron impact ionization mass spectrometry, is performed with a mass spectrometer having a mass accuracy of ≤ 5 parts per million (ppm), e.g. ≤ 1, 2, 2.5, 3, 4, or 5 ppm, and/or a mass resolution of between 10 000 and 100 000, preferably of between 20 000 and 100 000, more preferably of between 40 000 and 100 000, e.g. 40 000. The mass spectrometry, such as electron impact ionization mass spectrometry, may also be preformed with a mass spectrometer having a mass accuracy of 1-2 ppm and a mass resolution of 40 000. For example, as mass spectrometer, the MSD5975C XL quadrupole mass spectrometer of Agilent Technologies is used (see experimental section). Preferably, the mass spectrometry, such as electron impact ionization mass spectrometry, is performed with an upstream gas chromatography. It is preferred that the gas chromatography column used in gas chromatography (i) has a length of at least 20 m, preferably of at least 30 m, e.g. of 30 m, (ii) has an inner diameter of at least 0.15 mm, preferably of at least 0.25 mm, e.g. of 0.25 mm, and/or (iii) has a film thickness of at least 0.15 µm, preferably of at least 0.25 µm, e.g. of 0.25 µm. It is, alternatively or additionally, further preferred that (i) helium is used as a carrier gas, and/or (ii) the flow rate during gas chromatography is at least 0.5 ml/min, preferably is at least 1.0 ml/min, e.g. is 1.0 ml/min. For example, as gas chromatography column, a 30 m Agilent VF-5MS column with 0.25 mm inner diameter and 0.25 µm film thickness with a 10 m guard column of Agilent Technologies is used. For examples, as gas chromatograph, the gas chromatograph 7890A of Agilent Technologies is used, e.g. upstream of the above mentioned MSD5975C XL quadrupole mass spectrometer of Agilent Technologies (see experimental section).

As mentioned above, the metabolite Var878 is 2-oxo-glutaric acid, the metabolite Var62 is phosphoric acid, the metabolite Var37 is oxamide, and the metabolite Var19 is fumaric acid. Thus, in one preferred embodiment of the method of the present invention, the level of each one of the metabolites (i) Var878 and Var62, (ii) Var878 and Var37, (iii) Var878 and Var19, (iv) Var62 and Var37, (v) Var62 and Var19, (vi) Var37 and Var19, (vii) Var878, Var62, and Var37, (viii) Var878, Var62, and Var19, (ix) Var878, Var37 and Var19, (x) Var62, Var37, and Var19, or (xi) Var878, Var62, Var37, and Var19 is determined.

In one preferred embodiment of the method of the present invention, the level is a relative or an absolute concentration. Thus, it is preferred that the metabolite level which is determined in step (i) of the method of the present invention is a relative or absolute concentration. For example, the relative or absolute concentration of each one of at least two metabolites selected from the group consisting of Var878, Var62, Var37, and Var19 is determined in a sample obtained from a sugar beet seedling in step (i) of the method of the present invention. It is also preferred that the metabolite level which is used as a reference is a relative or absolute concentration, or that the metabolite levels which are used to train/obtain an algorithm or a mathematical function are relative or absolute concentrations. It is particularly preferred that the metabolite level which is determined in step (i) of the method of the present invention and the metabolite level which is used as a reference, or the metabolite levels which are used to train/obtain an algorithm or a mathematical function are identical, i.e. are either relative or absolute concentrations.

How such a concentration can be measured is known in the art, but also exemplarily described herein. For example, the relative concentration of a metabolite is indicated as a percentage value, e.g. per cent of the concentration of the metabolite, compared to an internal standard, e.g. diclofenac or 13C sorbitol. The internal standard can be added to the sample in an absolute concentration of between 100 and 600 µg/ml, e.g. of between 150 and 550 µg/ml, between 150 and 300 µg/ml, or between 400 and 600 µg/ml. For example, the internal standard diclofenac can be added to the sample in an absolute concentration of between 150 and 300 µg/ml, e.g. of between 150 and 200 µg/ml such as 188 µg/ml. For example, the internal standard 13C sorbitol can be added to the sample in an absolute concentration of between 400 and 600 µg/ml, e.g. of between 500 and 600 µg/ml such as 533 µg/ml. The absolute concentration of a metabolite is, for example, indicated as a concentration value, e.g. concentration in the tested sample, or absolute quantity of the metabolite, e.g. per tested sample. As a non-limiting example, the concentration of the metabolite to be employed in the context of the present invention is derived from the area of a peak corresponding to said metabolite and being obtained from, for example, chromatography, like gas chromatography (GC), preferably gas chromatography-mass spectrometry (GC-MS) or liquid chromatography (LC), preferably liquid chromatography-mass spectrometry (LC-MS).

As mentioned above, the level of each metabolite is compared to a reference in step (ii) of the method of the present invention, wherein said comparison allows predicting the sugar content in the full-grow sugar beet.

In one embodiment of the method of the present invention, the reference is the level (e.g. relative or absolute concentration) of said metabolite in sugar beet seedlings known to have
a high sugar content,
a middle sugar content, or
a low sugar content
in the resulting full-grown sugar beets.

As mentioned above, the reference is the level of said metabolite. This is to be understood that each metabolite has a corresponding reference level. In other words, different metabolites use/employ different reference levels. It is particularly preferred that said reference level is an average level of said metabolite which is determined by measuring the level of said metabolite in more than two, e.g. more than 5, 10, 50, 100, 179, 200, 400, 600, 800, 1000, 1500, 2000, 5000, 10 000, sugar beet seedlings. Said sugar beet seedlings may be from the same genotype or from different genotypes, e.g. from 2 to 20 different genotypes.

In one preferred embodiment of the method of the present invention, the reference is the relative concentration of said metabolite in sugar beet seedlings known to have
a high sugar content,
a middle sugar content, or
a low sugar content
in the resulting full-grown sugar beets. In other words, the relative concentration of said metabolite is correlated to the sugar content in the full-grown sugar beet. Preferably, the high sugar content represents a relative concentration of sugar of > 102 %, the middle sugar content represents a relative concentration of sugar of ≥ 97 to 102 %, or the low sugar content represents a relative concentration of sugar of < 97 %.

In one another preferred embodiment of the method of the present invention, the reference is the relative concentration of said metabolite in sugar beet seedlings known to have
a high sugar content, or
a low sugar content
in the resulting full-grown sugar beets. In other words, the relative concentration of said metabolite is correlated to the sugar content in the full-grown sugar beet. Preferably, the high sugar content represents a relative concentration of sugar of ≥ 100 %, or the low sugar content represents a relative concentration of sugar of < 100 %.

How such relative concentrations of sugar are determined, is known to the person skilled in the art, but also exemplarily described in the experimental section.

Alternatively, the known sugar content may represent an absolute concentration.

If, for example, the metabolite levels determined in step (i) of the method of the present invention are identically equal to the metabolite levels of the references representative for a high sugar content, the sugar beet may be predicted to have a high sugar content when full-grown, if, for example, the metabolite levels determined in step (i) of the method of the present invention are identically equal to the metabolite levels of the references representative for a middle sugar content, the sugar beet may be predicted to have a middle sugar content when full-grown, or if, for example, the metabolite levels determined in step (i) of the method of the present invention are identically equal to the metabolite levels of the references representative for a low sugar content, the sugar beet may be predicted to have a low sugar content when full-grown. Said levels may be relative concentrations or absolute concentrations.

In one alternative embodiment of the method of the present invention, the reference is the level (e.g. relative or absolute concentration) of said metabolite in sugar beet seedlings for which the sugar content in the resulting full-grown sugar beets is known.

As mentioned above, the reference is the level of said metabolite. This is to be understood that each metabolite has a corresponding reference level. In other words, different metabolites use/employ different reference levels (see above).

It is particularly preferred that said reference level is an average level of said metabolite which is determined by measuring the level of said metabolite in more than two, e.g. more than 5, 10, 50, 100, 179, 200, 400, 600, 800, 1000, 1500, 2000, 5000, 10 000, sugar beet seedlings. Said sugar beet seedlings may be from the same genotype or from different genotypes, e.g. from 2 to 20 different genotypes.

It is preferred that the levels of said metabolites are relative concentrations. The known sugar content may represent a relative concentration of sugar of between 70 % and 120 %, e.g. of 70, 71, 72, 73, 74, 75, 76, 77, 78, 79, 80, 81 ,82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 %. A known sugar content representing a relative concentration of sugar of ≥ 100 % may be indicative for a high sugar content, or a known sugar content representing a relative concentration of sugar of < 100 % may be indicative for a low sugar content.

How such relative concentrations of sugar are determined, is known to the person skilled in the art, but also exemplarily described in the experimental section.

Alternatively, the known sugar content may represent an absolute concentration.

If, for example, the metabolite levels determined in step (i) of the method of the present invention are identically equal to the metabolite levels of the references representative for a specific sugar content (see, for example, the relative concentrations of sugar mentioned above), the sugar beet may be predicted to have said specific sugar content when full-grown. Said levels may be relative concentrations or absolute concentrations.

As mentioned above, an algorithm or a mathematical function is applied to the levels of the metabolites in step (ii) of the method of the present invention, wherein said application allows predicting the sugar content in the full-grow sugar beet.

In one embodiment of the method of the present invention, the algorithm or mathematical function is obtained from/trained by
the levels (e.g. relative or absolute concentrations) of said metabolites in sugar beet seedlings for which the sugar content in the resulting full-grown sugar beets is known.

As mentioned above, the algorithm or mathematical function is obtained from/trained by the levels of said metabolites. In this respect, it should be noted that said metabolites correspond to the metabolites whose level is determined in step (i) of the method of the present invention.

It is particularly preferred that the levels of said metabolites are determined in more than two, e.g. more than 5, 10, 50, 100, 200, 400, 600, 800, 1000, 1500, 2000, 5000, 10 000, sugar beet seedlings. Said sugar beet seedlings may be from the same genotype or from different genotypes, e.g. from 2 to 20 different genotypes.

In one preferred embodiment of the method of the present invention, the algorithm or mathematical function is obtained using a machine learning approach. In one more preferred embodiment of the present invention, the machine learning approach involves the following steps:
(i) inputting the levels of said metabolites in sugar beet seedlings for which the sugar content in the resulting full-grown sugar beets is known, and
(ii) computing an algorithm or a mathematical function based on said levels that is suitable to predict the sugar content in the full-grown sugar beet.

It is preferred that the levels of said metabolites are relative concentrations. The known sugar content may represent a relative concentration of sugar of between 70 % and 120 %, e.g. of 70, 71, 72, 73, 74, 75, 76, 77, 78, 79, 80, 81 ,82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 %. A known sugar content representing a relative concentration of sugar of ≥ 100 % may be indicative for a high sugar content or a known sugar content representing a relative concentration of sugar of < 100 % may be indicative for a low sugar content.

How such relative concentrations of sugar are determined, is known to the person skilled in the art, but also exemplarily described in the experimental section.

How selected metabolites can be combined to form a predictive model of different accuracy and complexity is described in the experimental section (see paragraph "Regression model" and **Tables 3** and **4** of the experimental section).

Alternatively, the known sugar content may represent an absolute concentration.

In one alternative embodiment, the algorithm or mathematical function is obtained from/trained by the levels (e.g. relative or absolute concentrations) of said metabolites in sugar beet seedlings belonging to two or more categories of sugar content in the resulting full-grown sugar beets.

As mentioned above, the algorithm or mathematical function is obtained from/trained by the levels of said metabolites. In this respect, it should be noted that said metabolites correspond to the metabolites whose level is determined in step (i) of the method of the present invention.

It is particularly preferred that the levels of said metabolites are determined in more than two, e.g. more than 5, 10, 50, 100, 200, 400, 600, 800, 1000, 1500, 2000, 5000, 10 000, sugar beet seedlings. Said sugar beet seedlings may be from the same genotype or from different genotypes, e.g. from 2 to 20 different genotypes.

Preferably, the categories of sugar content in the resulting full-grown sugar beets are selected from the group consisting of a known low sugar content, a known middle sugar content and a known high sugar content.

Thus, in one preferred embodiment of the method of the present invention, the algorithm or mathematical function is obtained from/trained by
the levels of said metabolites in sugar beet seedlings known to have a high sugar content in the resulting full-grown sugar beets,
the levels of said metabolites in sugar beet seedlings known to have a middle sugar content in the resulting full-grown sugar beets, and
the levels of said metabolites in sugar beet seedlings known to have a low sugar content in the resulting full-grown sugar beets.
It is particularly preferred that the levels of said metabolites are relative concentrations and/or that the high sugar content represents a relative concentration of sugar of > 102 %, the middle sugar content represents a relative concentration of sugar of ≥ 97 to 102 %, and the low sugar content represents a relative concentration of sugar of < 97 %.

In one another preferred embodiment of the method of the present invention, the algorithm or mathematical function is obtained from/trained by
the levels of said metabolites in sugar beet seedlings known to have a high sugar content in the resulting full-grown sugar beets, and
the levels of said metabolites in sugar beet seedlings known to have a low sugar content in the resulting full-grown sugar beets.
It is particularly preferred that the levels of said metabolites are relative concentrations and/or that the high sugar content represents a relative concentration of sugar of ≥ 100 %, and/or the low sugar content represents a relative concentration of sugar of < 100 %.

In one more preferred embodiment of the method of the present invention, the algorithm or mathematical function is obtained using a machine learning approach. In one even more preferred embodiment, the machine learning approach involves the following steps:
(i) inputting the levels of said metabolites in sugar beet seedlings belonging to two or more categories of sugar content in the resulting full-grown sugar beets, and
(ii) computing an algorithm or a mathematical function based on said levels that is suitable to distinguish between the two or more categories of sugar content in the full-grown sugar beet.

Preferably, the categories of sugar content in the resulting full-grown sugar beets are selected from the group consisting of a known low sugar content, a known middle sugar content and a known high sugar content.

Thus, in one most preferred embodiment, the machine learning approach involves the following steps:
(i) inputting the levels of said metabolites in sugar beet seedlings known to have a high sugar content in the resulting full-grown sugar beets, the levels of said metabolites in sugar beet seedlings known to have a middle sugar content in the resulting full-grown sugar beets, and the levels of said metabolites in sugar beet seedlings known to have a low sugar content in the resulting full-grown sugar beet, and
(ii) computing an algorithm or a mathematical function based on said levels that is suitable to distinguish between the category of a high sugar content, the category of a middle sugar content, and the category of a low sugar content in the full-grown sugar beet.
It is particularly preferred that the levels of said metabolites are relative concentrations and/or that the high sugar content represents a relative concentration of sugar of > 102 %, the middle sugar content represents a relative concentration of sugar of ≥ 97 to 102 %, and/or the low sugar content represents a relative concentration of sugar of < 97 %.

In one another most preferred embodiment, the machine learning approach involves the following steps:
(i) inputting the levels of said metabolites in sugar beet seedlings known to have a high sugar content in the resulting full-grown sugar beets, and the levels of said metabolites in sugar beet seedlings known to have a low sugar content in the resulting full-grown sugar beets, and
(ii) computing an algorithm or a mathematical function based on said levels that is suitable to distinguish between the category of a high sugar content and the category of a low sugar content in the full-grown sugar beet.

It is particularly preferred that the levels of said metabolites are relative concentrations and/or that the high sugar content represents a relative concentration of sugar of ≥ 100 %, and/or the low sugar content represents a relative concentration of sugar of < 100 %.

How such relative concentrations of sugar are determined, is known to the person skilled in the art, but also exemplarily described in the experimental section.

If, for example, an algorithm or a mathematical function, which is obtained from
the levels of said metabolites in sugar beet seedlings know to have a high sugar content in the resulting full-grown sugar beets, and
the levels of said metabolites in sugar beet seedlings known to have a low sugar content in the resulting full-grow sugar beets,
is applied to the levels of the metabolites determined in step (i), the sugar beet is classified/predicted as having a high sugar content when full-grown, if the levels are above specified thresholds, and the sugar beet is classified/predicted as having a low sugar content when full-grown, if the levels are below specified thresholds. Said levels may be relative concentrations.

How selected metabolites can be combined to form a predictive model of different accuracy and complexity is described in the experimental section (see paragraph "Classification model" and **Tables 6** and **7** of the experimental section).

Alternatively, the known sugar content may represent an absolute concentration.

As mentioned above, it is preferred that the algorithm or mathematical function is obtained using a machine learning approach. Machine learning approaches may include, but are not limited to, supervised or unsupervised analysis: classification techniques (e.g. naïve Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal probit regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.

Support vector machines (SVMs) are a set of related supervised learning methods which are preferably used for classification and regression.

For example, given a set of training examples, each marked as belonging to one of two categories (e.g. high sugar content and low sugar content), an SVM algorithm builds a model that predicts whether a new example (e.g. sample to be tested) falls into one category or the other (e.g. high sugar content or low sugar content). A SVM model is a representation of the training examples as points in space, mapped so that the training examples of the separate categories (e.g. high sugar content or low sugar content) are divided by a clear gap that is as wide as possible. New examples (e.g. samples to be tested) are then mapped into that same space and predicted to belong to a category based on which side of the gap they fall on (e.g. high sugar content or low sugar content). More formally, a support vector machine constructs a hyperplane or set of hyperplanes in a high or infinite dimensional space, which can be used for classification, regression or other tasks. A good separation is achieved by the hyperplane that has the largest distance to the nearest training data points of any class (so-called functional margin), since in general the larger the margin the lower the generalization error of the classifier.

Classifying data is a preferred task in machine learning. For example, considering some given data points each belong to one (e.g. high sugar content or low sugar content) of two classes (e.g. high sugar content and low sugar content), the goal is to decide which class a new data point (e.g. achieved from a sample) will be in. In the preferred case of support vector machines, a data point is viewed as a p-dimensional vector (a list of p numbers), and the question is, whether it is possible to separate such points with a p-1-dimensional hyperplane. This is called a linear classifier. There are many hyperplanes that might classify the data. One reasonable choice as the best hyperplane is the one that represents the largest separation, or margin, between the two classes (e.g. condition of an acute coronary syndrome or no acute coronary syndrome). Thus, the hyperplane should be chosen so that the distance from it to the nearest data point on each side is maximized. If such a hyperplane exists, it is known as the maximum-margin hyperplane and the linear classifier it defines is known as a maximum margin classifier.

It is clear for the skilled person that the species of the sugar beet seedling whose metabolite level is determined by means of a sample in step (i) of the method of the present invention and the species of the full-grown sugar beet whose sugar content is predicted on the basis of this information with the method of the present invention are identical.

It is also preferred that the sugar beet whose metabolite level is used as a reference or whose metabolite levels are used to train/obtain an algorithm or a mathematical function are from sugar beets.

It is more preferred that the species of the sugar beet whose sugar content is predicted with the method of the present invention and the species of the sugar beets whose metabolite level is used as a reference or whose metabolite levels are used to train/obtain the algorithm or mathematical function are identical. It is even more preferred that the variety of the sugar beet whose sugar content is predicted with the method of the present invention and the variety of the sugar beets whose metabolite level is used as a reference or whose metabolite levels are used to train/obtain the algorithm or mathematical function are identical. The sugar beet whose sugar content is predicted with the method of the present invention and the sugar beets whose metabolite level is used as a reference or whose metabolite levels are used to train/obtain the algorithm or mathematical function may be from the same genotype or from different genotypes, e.g. from 2 to 20 different genotypes. It is particularly preferred that the sugar beet whose sugar content is predicted with the method of the present invention and the sugar beets whose metabolite level is used as a reference or whose metabolite levels are used to train/obtain the algorithm or mathematical function are grown under comparable conditions, for example, under comparable climatic conditions (e.g. in a glass house or in another protected environment), watering conditions, fertilizer conditions and/or ground conditions (e.g. soil, sand, or rock wool).

In one embodiment of the method of the present invention, the sample obtained from a sugar beet seedling is a leaf or stem.

In one another embodiment of the method of the present invention the sugar beet seedling is a seedling at the 2- to 8-leaf stage, e.g. at the 2-leaf stage, 3-leaf stage, 4-leaf stage, 5-leaft stage, 6-leaf stage, 7-leaf stage, or 8-leaf stage.

In one embodiment of the method of the present invention, the level, preferably the relative or absolute concentration, is determined by chromatography, spectrometry, or a combination thereof. This is to be understood that the level of each one of at least two metabolites selected from the group consisting of Var878, Var62, Var37, and Var19 is determined in a sample obtained from a sugar beet seedling by chromatography, spectrometry, or a combination thereof in step (i) of the method of the present invention. Preferably,
(i) the chromatography is liquid chromatography (LC), preferably reverse phase liquid chromatography (RP-LC), or gas chromatography (GC),
(ii) the spectrometry is mass spectrometry (MS), preferably tandem mass spectrometry (MS/MS), or
(iii) the chromatography is combined with spectrometry and is liquid chromatography-mass spectrometry (LC-MS) or gas chromatography-mass spectrometry (GC-MS).

The sample used in the method of the first aspect of present invention may have undergone chromatographic or other chemical processing before entering the mass spectrometer.

It is preferred that the mass spectrometry, is performed with a mass spectrometer having a mass accuracy of ≤ 5 parts per million (ppm), e.g. ≤ 1, 2, 2.5, 3, 4, or 5 ppm, and/or a mass resolution of between 10 000 and 100 000, preferably of between 20 000 and 100 000, more preferably of between 40 000 and 100 000, e.g. 40 000.

It is, additionally or alternatively, further preferred that the mass spectrometry is selected from the group consisting of an electrospray ionization mass spectrometry (ESI-MS), an electron impact ionization mass spectrometry (EI-MS), a matrix-assisted laser desorption/ionization mass spectrometry (MALDI-MS), an electron capture dissociation mass spectrometry (ECD-MS), a surface enhanced laser desorption ionization mass spectrometry (SELDI-MS), and an atmospheric pressure chemical ionisation mass spectrometry (APCI-MS).

For liquid chromatography, the Zorbax SB-Aq Rapid Resolution HD 2.1 x 50 mm 1.8 Micron reverse phase C18 column of Agilent Technologies may be used. Liquid chromatography may be performed with the 1290 high pressure liquid chromatography (HPLC) system of Agilent Technologies.

For gas chromatography, a 30 m Agilent VF-5MS column with 0.25 mm inner diameter and 0.25 µm film thickness with a 10 m guard column of Agilent Technologies may be used. Gas chromatography may be performed with the gas chromatograph 7890A of Agilent Technologies.

Mass spectrometry may be performed with the MSD5975C XL quadrupole mass spectrometer of Agilent Technologies, or with the UHD Accurate Mass Q-TOF MS 6540 mass spectrometer of Agilent Technologies.

Mass spectrometry may be performed with an upstream liquid or gas chromatograph/chromatography system. For example, mass spectrometry may be performed with the MSD5975C XL quadrupole mass spectrometer of Agilent Technologies and with the upstream gas chromatograph 7890A of Agilent Technologies, or mass spectrometry may be performed with the UHD Accurate Mass Q-TOF MS 6540 mass spectrometer of Agilent Technologies and with the upstream 1290 HPLC system of Agilent Technologies (see experimental section).

In a second aspect, the present invention relates to the use of at least two metabolites selected from the group consisting of Var878, Var62, Var37, and Var19 for predicting the sugar content in a full-grown sugar beet, wherein Var878, Var62, Var37, and Var19 are defined as in the method according to the first aspect of the present invention.

As mentioned above, the metabolite Var878 is 2-oxo-glutaric acid, the metabolite Var62 is phosphoric acid, the metabolite Var37 is oxamide, and the metabolite Var19 is fumaric acid. Thus, in one preferred embodiment, the metabolites (i) Var878 and Var62, (ii) Var878 and Var37, (iii) Var878 and Var19, (iv) Var62 and Var37, (v) Var62 and Var19, (vi) Var37 and Var19, (vii) Var878, Var62, and Var37, (viii) Var878, Var62, and Var19, (ix) Var878, Var37 and Var19, (x) Var62, Var37, and Var19, or (xi) Var878, Var62, Var37, and Var19 are used for predicting the sugar content in a full-grown sugar beet.

Also described herein but not encompassed by the present invention is the following:
1. Method for predicting the sugar content in a full-grown root vegetable comprising the steps of:
   (i) determining the level of each one of at least two metabolites selected from the group consisting of Var697, Var634, Var843, Var694, Var633, Var442, Var635, Var844, Var490, Var787, Var140, Var878, Var352, Var22, Var292, Var329, Var43, Var863, Var443, Var102, Var502, Var103, Var62, Var37, Var19, Var854, Var98, Var659, Var485, Var630, and Var124 in a sample obtained from a root vegetable seedling, wherein Var697 has an accurate mass of 470.1816 Da ± 3 parts per million (ppm), Var634 has an accurate mass of 474.1372 Da ± 3 ppm, Var843 has an accurate mass of 640.1631 Da ± 3 ppm, Var694 has an accurate mass of 245.9707 Da ± 3 ppm, Var633 has an accurate mass of 444.1265 Da ± 3 ppm, Var442 has an accurate mass of 148.0384 Da ± 3 ppm, Var635 has an accurate mass of 474.1155 Da ± 3 ppm, Var844 has an accurate mass of 608.1718 Da ± 3 ppm, Var490 has an accurate mass of 406.0365 Da ± 3 ppm, Var787 has an accurate mass of 785.5141 Da ± 3 ppm, Var140 has an accurate mass of 316.2015 Da ± 3 ppm, Var878 has an accurate mass of 146.0218 Da ± 3 ppm, Var352 has an accurate mass of 396.1409 Da ± 3 ppm, Var22 has a retention index of 1320.58 ± 2, Var292 has an accurate mass of 214.0093 Da ± 3 ppm, Var329 has an accurate mass of 1110.5208 Da ± 3 ppm, Var43 has a retention index of 2360.00 ± 2, Var863 has an accurate mass of 1433.9882 Da ± 3 ppm, Var443 has an accurate mass of 301.1275 Da ± 3 ppm, Var102 has an accurate mass of 286.9731 Da ± 3 ppm, Var502 has an accurate mass of 636.1681 Da ± 3 ppm, Var103 has an accurate mass of 192.0273 Da ± 3 ppm, Var62 has a retention index of 1262.34 ± 2, Var37 has a retention index of 1328.63 ± 2, Var19 has a retention index of 1347.49 ± 2, Var854 has an accurate mass of 822.5411 Da ± 3 ppm, Var98 has an accurate mass of 156.0036 Da ± 3 ppm, Var659 has an accurate mass of 884.4978 Da ± 3 ppm, Var485 has an accurate mass of 134.0218 Da ± 3 ppm, Var630 has an accurate mass of 245.9985 Da ± 3 ppm, and Var124 has an accurate mass of 208.0735 Da ± 3 ppm,
      wherein Var697 has a retention time of 2.732 min ± 0.25 min, Var634 has a retention time of 3.172 min ± 0.25 min, Var843 has a retention time of 3.660 min ± 0.25 min, Var694 has a retention time of 0.323 min ± 0.25 min, Var633 has a retention time of 3.151 min ± 0.25 min, Var442 has a retention time of 5.420 min ± 0.25 min, Var635 has a retention time of 3.655 min ± 0.25 min, Var844 has a retention time of 3.674 min ± 0.25 min, Var490 has a retention time of 0.549 min ± 0.25 min, Var787 has a retention time of 6.068 min ± 0.25 min, Var140 has a retention time of 5.293 min ± 0.25 min, Var878 has a retention time of 0.399 min ± 0.25 min, Var352 has a retention time of 3.947 min ± 0.25 min, Var292 has a retention time of 0.556 min ± 0.25 min, Var329 has a retention time of 4.870 min ± 0.25 min, Var863 has a retention time of 6.447 min ± 0.25 min, Var443 has a retention time of 0.540 min ± 0.25 min, Var102 has a retention time of 0.556 min ± 0.25 min, Var502 has a retention time of 3.453 min ± 0.25 min, Var103 has a retention time of 0.556 min ± 0.25 min, Var854 has a retention time of 5.999 min ± 0.25 min, Var98 has a retention time of 0.423 min ± 0.25 min, Var659 has a retention time of 6.213 min ± 0.25 min, Var485 has a retention time of 0.381 min ± 0.25 min, Var630 has a retention time of 0.405 min ± 0.25 min, and Var124 has a retention time of 4.005 min ± 0.25 min in liquid chromatography (LC), and
      wherein Var22 shows fragments having a nominal mass of 292, 189, 307, 205, and 133 Da, Var43 shows fragments having a nominal mass of 375, 292, 305, 204, and 217 Da, Var62 shows fragments having a nominal mass of 314, 299, 211, 283, and 225 Da, Var37 shows fragments having a nominal mass of 102, 189, 117, 217, and 131 Da, and Var19 shows fragments having a nominal mass of 245, 115, 217, 143, and 133 Da in mass spectrometry (MS); and
   (ii) comparing the level of each metabolite to a reference, wherein said comparison allows predicting the sugar content in the full-grown root vegetable, and/or
      applying an algorithm or a mathematical function to the levels of the metabolites, wherein said application allows predicting the sugar content in the full-grown root vegetable.
2. The method of item 1, wherein the level of each one of at least two metabolites selected from the group consisting of Var697, Var634, Var843, Var694, Var633, Var442, Var635, Var844, Var490, Var787, Var140, Var878, Var352, Var22, Var292, Var329, Var43, Var863, Var443, and Var102 is determined.
3. The method of item 2, wherein the level of each one of the metabolites
   (i) Var697 and Var634,
   (ii) Var697, Var634, Var843, and Var694,
   (iii) Var697, Var634, Var843, Var694, Var633, Var442, Var635, and Var844,
   (iv) Var697, Var634, Var843, Var694, Var633, Var442, Var635, Var844, Var490, Var787, Var140, Var878, and Var352,
   (v) Var697, Var634, Var843, Var694, Var633, Var442, Var635, Var844, Var490, Var787, Var140, Var878, Var352, Var22, Var292, Var329, and Var43, or
   (vi) Var697, Var634, Var843, Var694, Var633, Var442, Var635, Var844, Var490, Var787, Var140, Var878, Var352, Var22, Var292, Var329, Var43, Var863, Var443, and Var102
   is determined.
4. The method of item 1, wherein the level of each one of at least two metabolites selected from the group consisting of Var697, Var843, Var490, Var502, Var103, Var22, Var62, Var37, Var19, Var854, Var98, Var659, Var485, Var630, Var634, and Var124 is determined.
5. The method of item 4, wherein the level of each one of the metabolites
   (i) Var697 and Var843,
   (ii) Var697, Var843, Var490, Var502, and Var103,
   (iii) Var697, Var843, Var490, Var502, Var103, Var22, Var62, Var37, and Var19,
   (iv) Var697, Var843, Var490, Var502, Var103, Var22, Var62, Var37, Var19, Var854, Var98, Var659, and Var485, or
   (v) Var697, Var843, Var490, Var502, Var103, Var22, Var62, Var37, Var19, Var854, Var98, Var659, Var485, Var630, Var634, and Var124
   is determined.
6. The method of any one of items 1 to 5, wherein the reference is the level of said metabolite in root vegetable seedlings known to have a high sugar content, a middle sugar content, or a low sugar content in the resulting full-grown root vegetables.
7. The method of any one of items 1 to 5, wherein the reference is the level of said metabolite in root vegetable seedlings for which the sugar content in the resulting full-grown root vegetables is known.
8. The method of any one of items 1 to 7, wherein the algorithm or mathematical function is obtained from the levels of said metabolites in root vegetable seedlings for which the sugar content in the resulting full-grown root vegetables is known.
9. The method of any one of items 1 to 7, wherein the algorithm or mathematical function is obtained from the levels of said metabolites in root vegetable seedlings belonging to two or more categories of sugar content in the resulting full-grown root vegetables.
10. The method of item 9, wherein the categories of sugar content in the resulting full-grown root vegetables are selected from the group consisting of a known low sugar content, a known middle sugar content and a known high sugar content.
11. The method of item 10, wherein the algorithm or mathematical function is obtained from the levels of said metabolites in root vegetable seedlings known to have a high sugar content in the resulting full-grown root vegetables, and the levels of said metabolites in root vegetable seedlings known to have a low sugar content in the resulting full-grown root vegetables.
12. The method of any one of items 1 to 11, wherein the algorithm or mathematical function is obtained using a machine learning approach.
13. The method of item 12, wherein the machine learning approach involves the following steps:
   (i) inputting the levels of said metabolites in root vegetable seedlings for which the sugar content in the resulting full-grown root vegetables is known, and
   (ii) computing an algorithm or a mathematical function based on said levels that is suitable to predict the sugar content in the full-grown root vegetable.
14. The method of item 12, wherein the machine learning approach involves the following steps:
   (i) inputting the levels of said metabolites in root vegetable seedlings belonging to two or more categories of sugar content in the resulting full-grown root vegetables, and
   (ii) computing an algorithm or a mathematical function based on said levels that is suitable to distinguish between the two or more categories of sugar content in the full-grown root vegetable.
15. The method of item 14, wherein the categories of sugar content in the resulting full-grown root vegetables are selected from the group consisting of a known low sugar content, a known middle sugar content and a known high sugar content.
16. The method of item 15, wherein the machine learning approach involves the following steps:
   (i) inputting the levels of said metabolites in root vegetable seedlings known to have a high sugar content in the resulting full-grown root vegetables and the levels of said metabolites in root vegetable seedlings known to have a low sugar content in the resulting full-grown root vegetables, and
   (ii) computing an algorithm or a mathematical function based on said levels that is suitable to distinguish between the category of a high sugar content and the category of a low sugar content in the full-grown root vegetable.
17. The method of any one of items 1 to 16, wherein the root vegetable(s) is (are) selected from the group consisting of (a) beet(s), carrot(s), parsnip(s), crummock(s), and potato(es).
18. The method of item 17, wherein
   (i) the beet(s) is (are) selected from the group consisting of (a) sugar beet(s), forage beet(s), beetroot(s), and turnip(s), or
   (ii) the potato(es) is (are) selected from the group consisting of (a) sweet potato(es) and table potato(es).
19. The method of any one of items 1 to 18, wherein the mass spectrometry is performed with a mass spectrometer having a mass accuracy of ≤ 5 parts per million (ppm) and a mass resolution of 40 000.
20. The method of any one of items 1 to 19, wherein the liquid chromatography (LC) is a reverse phase liquid chromatography (RP-LC).
21. The method of item 20, wherein the reverse phase liquid chromatography (RP-LC) is performed with the solvents water and methanol with 0.1 % formic acid and/or a silica-based stationary phase, preferably an octadecyl carbon chain (C18)-bonded silica-based stationary phase.
22. The method of any one of items 1 to 21, wherein the sample obtained from a root vegetable seedling is a leaf or stem.
23. The method of any one of items 1 to 22, wherein root vegetable seedling is a seedling at the 2- to 8-leaf stage.
24. The method of any one of items 1 to 23, wherein the level is determined by chromatography, spectrometry, or a combination thereof.
25. The method of item 24, wherein
   (i) the chromatography is liquid chromatography (LC), preferably reverse phase liquid chromatography (RP-LC), or gas chromatography (GC),
   (ii) the spectrometry is mass spectrometry (MS), preferably tandem mass spectrometry (MS/MS), or
   (iii) the chromatography is combined with spectrometry and is liquid chromatography-mass spectrometry (LC-MS) or gas chromatography-mass spectrometry (GC-MS).
26. The method of item 25, wherein the mass spectrometry is selected from the group consisting of an electrospray ionization mass spectrometry (ESI-MS), an electron impact ionization mass spectrometry (EI-MS), a matrix-assisted laser desorption/ionization mass spectrometry (MALDI-MS), an electron capture dissociation mass spectrometry (ECD-MS), a surface enhanced laser desorption/ionization mass spectrometry (SELDI-MS), and an atmospheric pressure chemical ionisation mass spectrometry (APCI-MS).
27. The method of any one of items 1 to 26, wherein the level is a relative or an absolute concentration.
28. Use of at least two metabolites selected from the group consisting of Var697, Var634, Var843, Var694, Var633, Var442, Var635, Var844, Var490, Var787, Var140, Var878, Var352, Var22, Var292, Var329, Var43, Var863, Var443, Var102, Var502, Var103, Var62, Var37, Var19, Var854, Var98, Var659, Var485, Var630, and Var124 for predicting the sugar content in a full-grown root vegetable.
29. The use of item 28, wherein the at least two metabolites are selected from the group consisting of Var697, Var634, Var843, Var694, Var633, Var442, Var635, Var844, Var490, Var787, Var140, Var878, Var352, Var22, Var292, Var329, Var43, Var863, Var443, and Var102.
30. The use of item 28, wherein the at least two metabolites are selected from the group consisting of Var697, Var843, Var490, Var502, Var103, Var22, Var62, Var37, Var19, Var854, Var98, Var659, Var485, Var630, Var634, and Var124.

### EXAMPLES

The examples given below are for illustrative purposes only and do not limit the invention described above in any way.

### 1. Cultivation

Seedlings were grown in soil from plant seeds of 20 different genotypes. The seedlings were cultured in a glass house or other protected environment. Leaf or stem of 179 seedlings were harvested and immediately shock frozen in liquid nitrogen.

### 2. Extraction

Sugar beet leaf tissue (150mg) was homogenised using a ball mill pre-cooled with liquid nitrogen and extracted in 1400µl of methanol, 60µl of internal standard (0.2mg ribitol µl⁻¹ water) was subsequently added as a quantification standard. The mixture was extracted for 15min at 70°C and mixed vigorously with 1 volume of water. After centrifugation at 2200g 250µL was taken and reduced to dryness *in vacuo* for GC-MS analysis. 200µL were taken for LC-MS analysis.

### 3. Analytical Setup

Metabolite extracts were measured on GC-MS and LC-MS in parallel. Metabolite analysis by GC-MS was carried out by a method modified from that described by Roessner et al. (2000) [3]. The Gas Chromatography-Mass spectrometry (GC-MS) system used comprised a Gerstel MPS2 autosampler, an Agilent gas chromatograph 7890A and an Agilent MSD5975C XL quadrupole mass spectrometer (Agilent Technologies, Waldbronn, Germany). The mass spectrometer was tuned according to the manufacturers' recommendations and had a mass accuracy of 1-2ppm. Gas chromatography was performed on a 30m Agilent VF-5MS column with 0.25mm inner diameter and 0.25µm film thickness with a 10m guard column (Agilent Technologies, Waldbronn, Germany). The injection temperature was set at 230°C, the interface at 250°C and the ion source adjusted to 200°C. Helium was used as the carrier gas at a flow rate of 1ml/min⁻¹. The analysis was performed under the following temperature program: 5min isothermal heating at 70°C, followed by a 5°C min⁻¹ oven temperature ramp to 350°C and a final 5min heating at 330°C. The system was then temperature equilibrated for 1 min at 70°C prior to injection of the next sample. Mass spectra were recorded at 3 scan s⁻¹ with an *m*/*z* 50-600 scanning range. Both chromatograms and mass spectra were evaluated using the TagFinder software (Metabolomic Discoveries GmbH, Potsdam, Germany). Retention time was normalized by applying the Kovats retention index system [4].

The Liquid Chromatography-Mass Spectrometry (LC-MS) system used comprised an Agilent UHD Accurate Mass Q-TOF MS 6540 with an accuracy of up to 2 ppm and a resolution of mass/Δmass = 40.000. The mass spectrometer was coupled to a 1290 HPLC system (Agilent Technologies, Waldbronn, Germany). Liquid chromatography was performed using a Zorbax SB-Aq Rapid Resolution HD 2.1 x 50 mm 1.8 Micron reverse phase C18 column (Agilent Technologies, Waldbronn, Germany) at 0.4ml/min. Eluent A was water and eluent B was methanol with 0.1 % formic acid (see **Table 1).**

**Table 1: Chromatographic conditions of performed liquid chromatography.**

| **Time [min]** | **Eluent A [%]** | **Eluent B [%]** |
|---|---|---|
| 1 | 100 | 0 |
| 5 | 10 | 90 |
| 5.5 | 5 | 95 |
| 6.5 | 5 | 95 |
| 6.51 | 0 | 100 |
| 8 | 0 | 100 |

The mass spectrometer was tuned according to the manufacturer's recommendations. The mass spectrometer was run in positive and negative mode with electrospray ionisation and a scanning mode from 50-1700Da. LC-MS data was analysed by the vendor software Agilent Mass Hunter version B05.00 (Agilent Technologies, Waldbronn, Germany).

### 4. Normalisation

GC-MS data was normalised by blank subtraction and total ion count (TIC). LC-MS data was normalised by the internal standard diclofenac and blank subtraction. All data was divided by gram fresh weight of the plant material. Metabolites with >20% missing values were filtered out. The remaining 900 metabolites were normalised on their median for each day of measurement as described by Lisec *et al* [5]. Missing values were replaced by an artificial value of 0.7 times the lowest detected value.

### 5. Statistical Analysis

### Sugar content

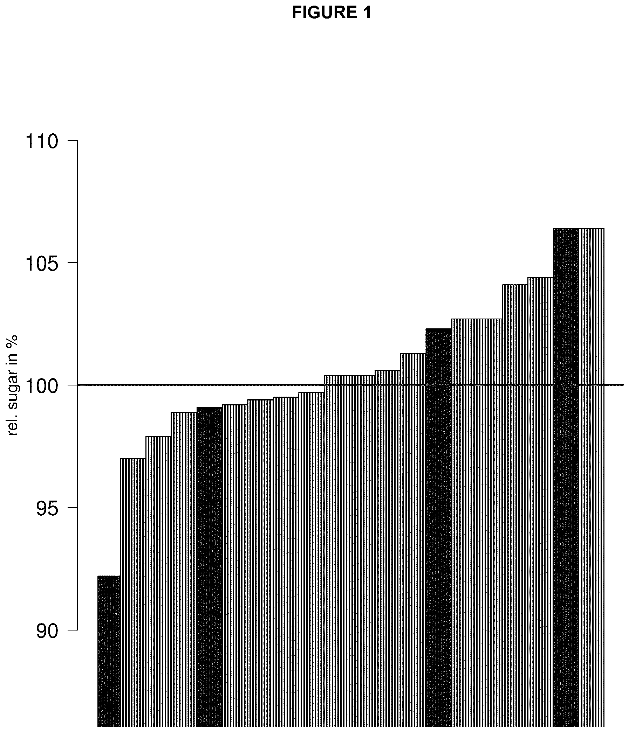

For all 20 investigated genotypes, the sugar content had been determined previously. The mean was calculated by four sugar beet lines: the highest, the lowest and two average sugar content lines. This mean acted as reference. All measured sugar contents were normalised by dividing by reference and multiplying with 100%. This resulted in relative sugar contents ranging from 92.2% to 106.4% (see **Figure 1**).

### Regression model

To select the metabolites that predict relative sugar content, a Random Forest [6] regression model was built using the R-package *randomForest* [7] [8]. 5000 trees were grown to create the final model. First, each tree was grown from a bootstrap sample consisting of two third of all samples, subsequently the tree was validated on the remaining third of all samples, the so called 'out-of-bag'-samples. Thus, the trees were internally validated: the overall mean of squared residuals (MSR) was 6.73.

During model generation, the Random Forest importance was calculated for each metabolite. This importance measure was based on the increasing mean squared error (MSE) for the out-of-bag portion for each tree: The difference in MSE using the true predictor as well as a permuted predictor variable was calculated for each tree, averaged over all trees, normalised and thus resulted in the importance measure.

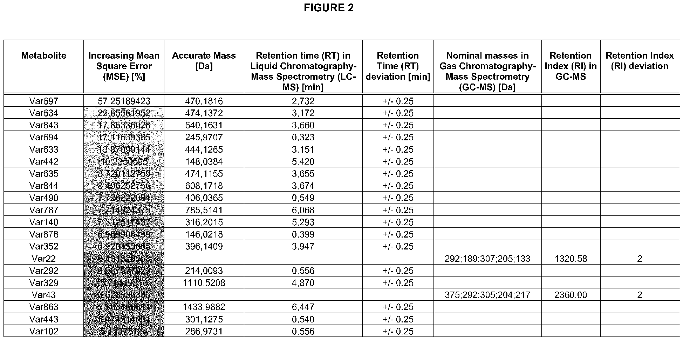

All metabolites with an importance above 5% were chosen to be relevant and split into five groups of increasing relevance (see **Table 2**). The selected metabolites are specified in **Figure 2****.**

**Table 2: Metabolites with an importance > 5% were selected, whereas the importance was defined as the % increase of the mean squared error (MSE). Those most important metabolites were divided into five subgroups of decreasing importance and increasing number of metabolites.**

| **Increasing Mean Squared Error (MSE)** | **Number of metabolites** |
|---|---|
| >30.0 | 1 |
| >20.0 | 3 |
| >8.0 | 4 |
| >6.5 | 5 |
| >5.0 | 7 |

The selected metabolites can be combined to form predictive models of different accuracy and complexity. As an example for such predictive models, a recursive partitioning approach was applied using the R-package *rpart* [9], which is based on the CART algorithm [10].

A simple model with low complexity and a root mean squared error (RMSE) of 2.35 is shown in **Table 3.**

**Table 3: Decision rules and predicted averaged sugar values as a result of the application of a recursive partitioning algorithm. The rules were limited to the use of two metabolites.**

| **Decision Rule** | **Predicted releative sugar content [%]** |
|---|---|
| Var697>=0.7747and Var329< 0.5185 | 95.7 |
| Var697>=0.7747and Var329>=0.5185 | 99.4 |
| Var697< 0.7747 and Var442>=0.499 | 101.9 |
| Var697< 0.7747 and Var442< 0.499 | 105.4 |

The selected metabolites can also be combined to more complex prediction models, as shown in **Table 4.** This second example had a RMSE of 1.62.

**Table 4: Decision rules and predicted averaged sugar values as a result of the application of a recursive partitioning algorithm. The rules were limited to the use of two, three, four, five, and six metabolites.**

| **Decision Rule** | **Predicted releative sugar content [%]** |
|---|---|
| Var697>=0.7746712 and Var694< 0.64422 | 94.0 |
| Var697>=0.7746712 and Var694>=0.64422 and Var490< 0.7487003 and Var22>=0.4825302 and Var635>=1.176211 | 96.5 |
| Var697>=0.7746712 and Var694>=0.64422 and Var490< 0.7487003 and Var22>=0.4825302 and Var635< 1.176211 | 98.4 |
| Var697>=0.7746712 and Var694>=0.64422 and Var490< 0.7487003 and Var22< 0.4825302 | 100.1 |
| Var697>=0.7746712 and Var694>=0.64422 and Var490>=0.7487003 | 100.3 |
| Var697< 0.7746712 and Var442>=0.4990274 and Var844>=1.90632 and Var22< 1.0 | 97.4 |
| Var697< 0.7746712 and Var442>=0.4990274 and Var844>=1.90632 and Var22>=1.0 | 100.6 |
| Var697< 0.7746712 and Var442>=0.4990274 and Var844< 1.90632 and Var635< 0.6107333 | 100.9 |
| Var697< 0.7746712 and Var442>=0.4990274 and Var844< 1.90632 and Var635>=0.6107333 and Var634< 1.222208 | 102.2 |
| Var697< 0.7746712 and Var442>=0.4990274 and Var844< 1.90632 and Var635>=0.6107333 and Var634>=1.222208 and Var442>=1.202274 | 102.3 |
| Var697< 0.7746712 and Var442>=0.4990274 and Var844< 1.90632 and Var635>=0.6107333 and Var634>=1.222208 and Var442< 1.202274 | 104.7 |
| Var697< 0.7746712 and Var442< 0.4990274 and Var878>=0.9876927 | 103.6 |
| Var697< 0.7746712 and Var442< 0.4990274 and Var878< 0.9876927 | 106.4 |

### Classification model

In addition, the prediction was simplified by transferring a regression into a two-class classification problem. For this purpose, the relative sugar content was split into two groups: low sugar content (< 100%, *i.e.* below average) and high sugar content (≥ 100%, *i.e.* above average). The group with low sugar content contained 80 samples, whereas the high sugar content group consisted of 99 samples.

A Random Forest classification model was built by growing 5000 trees. The out-of-bag estimate of the error rate of this model was 34.6%. The class error predicting the low sugar content samples was 55.0%, and the class error predicting the high sugar content samples was 18.2%.

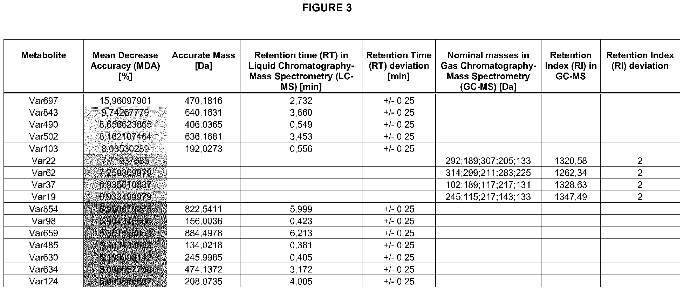

The importance measure for each metabolite was based on the mean decrease in accuracy when permuting the predictor variable in the out-of bag portion of the data. All metabolites with an importance above 5% were chosen to be relevant and split into four groups of increasing relevance (see **Table 5**). The selected metabolites are specified in **Figure 3****.**

**Table 5: Metabolites with an importance > 5% were selected, whereas the importance was defined as the percentage of increase of the mean squared error (MSE). Those most important metabolites were divided into five subgroups of decreasing importance and increasing number of metabolites.**

| **Mean Decrease Accuracy (MDA)** | **Number of metabolites** |
|---|---|
| >10.0 | 1 |
| >8.0 | 4 |
| >6.0 | 4 |
| >5.0 | 7 |

This selection can be combined to prediction models of varying accuracy and complexity. Again the decision rules were created by applying the recursive partitioning algorithm. The simple model in **Table 6** had a correct classification rate of 78%.

**Table 6: Decision rules and predicted category of sugar content as a result of the application of a recursive partitioning algorithm. The rules were limited to the use of two metabolites.**

| **Decision Rule** | **Predicted category of sugar content** |
|---|---|
| Var697>=0.7746712 and Var490< 0.8923371 | low |
| Var697>=0.7746712 and Var490>=0.8923371 | high |
| Var697< 0.7746712 and Var502>=2.466463 | low |
| Var697< 0.7746712 and Var502< 2.466463 | high |

The more complex model in **Table 7** had a correct classification rate of 86%.

**Table 7: Decision rules and predicted category of sugar content as a result of the application of a recursive partitioning algorithm. The rules were limited to the use of two, three, four, and five metabolites.**

| **Decision Rule** | **Predicted category of sugar content** |
|---|---|
| Var697>=0.7746712 and Var490< 0.8923371 | low |
| Var697>=0.7746712 and Var490>=0.8923371 and Var124< 0.709806 | low |
| Var697>=0.7746712 and Var490>=0.8923371 and Var124>=0.709806 and Var854< 1.088097 and Var37< 1.37732 | low |
| Var697>=0.7746712 and Var490>=0.8923371 and Var124>=0.709806 and Var854< 1.088097 and Var37>=1.37732 | high |
| Var697>=0.7746712 and Var490>=0.8923371 and Var124>=0.709806 and Var854>=1.088097 | high |
| Var697< 0.7746712 and Var502>=2.466463 | low |
| Var697< 0.7746712 and Var502< 2.466463 and Var634< 0.5222044 | low |
| Var697< 0.7746712 and Var502< 2.466463 and Var634>=0.5222044 | high |

### REFERENCES

[1] Kovats, E. (1958). "Gas-chromatographische Charakterisierung organischer Verbindungen. Teil 1: Retentionsindices aliphatischer Halogenide, Alkohole, Aldehyde und Ketone". Helv. Chim. Acta 41 (7): 1915-32. doi:10.1002/hlca.19580410703.
[2] IUPAC, Compendium of Chemical Terminology, 2nd ed. (the "Gold Book") (1997). Online corrected version: (2006-) "retention index, I in column chromatography".
[3] Roessner, Ute et al. "Simultaneous analysis of metabolites in potato tuber by gas chromatography-mass spectrometry." The Plant Journal 23.1 (2000): 131-142.
[4] Kovats, E. "Gas chromatographic characterization of organic compounds. I. Retention indexes of aliphatic halides, alcohols, aldehydes, and ketones." Helv. Chim. Acta 41 (1958): 1915-1932.
[5] Lisec J, Schauer N, Kopka J, Willmitzer L, Fernie AR. "Gas chromatography mass spectrometry-based metabolite profiling in plants." Nat Protoc. 2006; 1: 387-396. doi: 10.1038/nprot.2006.59.
[6] Breiman L. Random Forests. Mach Learn. 2001; 45: 5-32. doi: 10.1023/A:1010933404324.
[7] R Core Team (2013). R: "A language and environment for statistical computing." R Foundation for Statistical Computing, Vienna, Austria. ISBN 3-900051-07-0.
[8] Liaw A, Wiener M. "Classification and Regression by randomForest." R News. 2002; 2(3): 18-22.
[9] T.M Therneau and E.J Atkinson. "An introduction to recursive partitioning using the rpart routines." Division of Biostatistics 61, Mayo Clinic, 1997.
[10] Breiman, J.H. Friedman, R.A. Olshen, and C.J Stone. "Classification and Regression Trees." Wadsworth, Belmont, Ca, 1983.

## Claims

1. Method for predicting the sugar content in a full-grown sugar beet comprising the steps of:
(i) determining the level of each one of at least two metabolites selected from the group consisting of Var878, Var62, Var37, and Var19 in a sample obtained from a sugar beet seedling, wherein
Var878 is 2-oxo-glutaric acid, has an accurate mass of 146.0218 Da ± 3 ppm, and a retention time of 0.399 min ± 0.25 min in liquid chromatography (LC), Var62 is phosphoric acid, has a retention index of 1262.34 ± 2, and shows fragments having a nominal mass of 314, 299, 211, 283, and 225 Da in mass spectrometry (MS),
Var37 is oxamide, has a retention index of 1328.63 ± 2, and shows fragments having a nominal mass of 102, 189, 117, 217, and 131 Da in mass spectrometry (MS),
Var19 is fumaric acid, has a retention index of 1347.49 ± 2, and shows fragments having a nominal mass of 245, 115, 217, 143, and 133 Da in mass spectrometry (MS); and
(ii) comparing the level of each metabolite to a reference, wherein said comparison allows predicting the sugar content in the full-grown sugar beet, and/or applying an algorithm or a mathematical function to the levels of the metabolites, wherein said application allows predicting the sugar content in the full-grown sugar beet.

2. The method of claim 1, wherein the level of each one of the metabolites
(i) Var878 and Var62,
(ii) Var878 and Var37,
(iii) Var878 and Var19,
(iv) Var62 and Var37,
(v) Var62 and Var19,
(vi) Var37 and Var 19,
(vii) Var878, Var62, and Var37,
(viii) Var878, Var62, and Var19,
(ix) Var878, Var37, and Var19,
(x) Var62, Var37, and Var19, or
(xi) Var878, Var62, Var37, and Var19
is determined.

3. The method of any one of claims 1 or 2, wherein the reference is the level of said metabolite in sugar beet seedlings known to have
a high sugar content,
a middle sugar content, or
a low sugar content
in the resulting full-grown sugar beets.

4. The method of any one of claims 1 or 2, wherein the reference is the level of said metabolite in sugar beet seedlings for which the sugar content in the resulting full-grown sugar beets is known.

5. The method of any one of claims 1 to 4, wherein the algorithm or mathematical function is obtained from
the levels of said metabolites in sugar beet seedlings for which the sugar content in the resulting full-grown sugar beets is known.

6. The method of any one of claims 1 to 4, wherein the algorithm or mathematical function is obtained from
the levels of said metabolites in sugar beet seedlings belonging to two or more categories of sugar content in the resulting full-grown sugar beets.

7. The method of claim 6, wherein the categories of sugar content in the resulting full-grown sugar beets are selected from the group consisting of a known low sugar content, a known middle sugar content and a known high sugar content.

8. The method of claim 7, wherein the algorithm or mathematical function is obtained from the levels of said metabolites in sugar beet seedlings known to have a high sugar content in the resulting full-grown sugar beets, and
the levels of said metabolites in sugar beet seedlings known to have a low sugar content in the resulting full-grown sugar beets.

9. The method of any one of claims 1 to 8, wherein the algorithm or mathematical function is obtained using a machine learning approach.

10. The method of claim 9, wherein the machine learning approach involves the following steps:
(i) inputting the levels of said metabolites in sugar beet seedlings for which the sugar content in the resulting full-grown sugar beets is known, and
(ii) computing an algorithm or a mathematical function based on said levels that is suitable to predict the sugar content in the full-grown sugar beet.

11. The method of claim 9, wherein the machine learning approach involves the following steps:
(i) inputting the levels of said metabolites in sugar beet seedlings belonging to two or more categories of sugar content in the resulting full-grown sugar beets, and
(ii) computing an algorithm or a mathematical function based on said levels that is suitable to distinguish between the two or more categories of sugar content in the full-grown sugar beet.

12. The method of claim 11, wherein the categories of sugar content in the resulting full-grown sugar beets are selected from the group consisting of a known low sugar content, a known middle sugar content and a known high sugar content.

13. The method of claim 12, wherein the machine learning approach involves the following steps:
(i) inputting the levels of said metabolites in sugar beet seedlings known to have a high sugar content in the resulting full-grown sugar beets and the levels of said metabolites in sugar beet seedlings known to have a low sugar content in the resulting full-grown sugar beets, and
(ii) computing an algorithm or a mathematical function based on said levels that is suitable to distinguish between the category of a high sugar content and the category of a low sugar content in the full-grown sugar beet.

14. Use of at least two metabolites selected from the group consisting of Var878, Var62, Var37, and Var19 for predicting the sugar content in a full-grown sugar beet, wherein Var878, Var62, Var37, and Var19 are defined as in claim 1.

## Patentansprüche

1. Verfahren zum Vorhersagen des Zuckergehalts in einer voll ausgewachsenen Zuckerrübe, umfassend die folgenden Schritte:
(i) Bestimmen des Levels jedes der mindestens zwei Metaboliten, die aus der Gruppe ausgewählt sind, die aus Var878, Var62, Var37 und Var19 besteht, in einer Probe, die aus einem Zuckerrübenkeimling erhalten wurde, wobei
Var878 2-Oxoglutarsäure ist, eine genaue Masse von 146,0218 Da ± 3 ppm und eine Retentionszeit von 0,399 min ± 0,25 min in der Flüssigchromatographie (LC) aufweist,
Var62 Phosphorsäure ist, einen Retentionsindex von 1262,34 ± 2 aufweist und Fragmente mit einer nominalen Masse von 314, 299, 211, 283 und 225 Da in der Massenspektrometrie (MS) zeigt,
Var37 Oxamid ist, einen Retentionsindex von 1262,34 ± 2 aufweist und Fragmente mit einer nominalen Masse von 102, 189, 117, 217 und 131 Da in der Massenspektrometrie (MS) zeigt, Var19 Fumarsäure ist, einen Retentionsindex von 1262,34 ± 2 aufweist und Fragmente mit einer nominalen Masse von 245, 115, 217, 143 und 133 Da in der Massenspektrometrie (MS) zeigt; und
(ii) Vergleichen des Levels jedes Metaboliten mit einer Referenz, wobei das Vergleichen ein Vorhersagen des Zuckergehalts in der voll ausgewachsenen Zuckerrübe ermöglicht, und/oder Anwenden eines Algorithmus oder einer mathematischen Funktion auf die Level der Metaboliten, wobei die Anwendung ein Vorhersagen des Zuckergehalts in der ausgewachsenen Zuckerrübe ermöglicht.

2. Verfahren nach Anspruch 1, wobei das Level jedes der Metaboliten
(i) Var878 und Var62,
(ii) Var878 und Var37,
(iii) Var878 und Var19,
(iv) Var62 und Var37,
(v) Var62 und Var19,
(vi) Var37 und Var19,
(vii) Var878, Var62 und Var37,
(viii) Var878, Var62 und Var19,
(ix) Var878, Var37 und Var19,
(x) Var62, Var37 und Var19, oder
(xi) Var878, Var62, Var37 und Var19
bestimmt wird.

3. Verfahren nach einem der Ansprüche 1 oder 2, wobei die Referenz das Level des Metaboliten in Zuckerrübenkeimlingen ist, von denen bekannt ist, dass sie einen hohen Zuckergehalt, einen mittleren Zuckergehalt oder einen niedrigen Zuckergehalt in den resultierenden voll ausgewachsenen Zuckerrüben aufweisen.

4. Verfahren nach einem der Ansprüche 1 oder 2, wobei die Referenz das Level des Metaboliten in Zuckerrübenkeimlingen ist, für die der Zuckergehalt in den resultierenden voll ausgewachsenen Zuckerrüben bekannt ist.

5. Verfahren nach einem der Ansprüche 1 bis 4, wobei der Algorithmus oder die mathematische Funktion aus den Leveln der Metaboliten in Zuckerrübenkeimlingen erhalten wird, für die der Zuckergehalt in den resultierenden voll ausgewachsenen Zuckerrüben bekannt ist.

6. Verfahren nach einem der Ansprüche 1 bis 4, wobei der Algorithmus oder die mathematische Funktion aus den Leveln der Metaboliten in Zuckerrübenkeimlingen erhalten wird, die zu zwei oder mehr Kategorien von Zuckergehalt in den resultierenden voll ausgewachsenen Zuckerrüben gehören.

7. Verfahren nach Anspruch 6, wobei die Kategorien von Zuckergehalt in den resultierenden voll ausgewachsenen Zuckerrüben aus der Gruppe ausgewählt sind, die aus einem bekannten niedrigen Zuckergehalt, einem bekannten mittleren Zuckergehalt und einem bekannten hohen Zuckergehalt besteht.

8. Verfahren nach Anspruch 7, wobei der Algorithmus oder die mathematische Funktion aus den Leveln der Metaboliten in Zuckerrübenkeimlingen, von denen bekannt ist, dass sie einen hohen Zuckergehalt in den resultierenden voll ausgewachsenen Zuckerrüben aufweisen, und aus den Leveln der Metaboliten in Zuckerrübenkeimlingen, von denen bekannt ist, dass sie einen niedrigen Zuckergehalt in den resultierenden voll ausgewachsenen Zuckerrüben aufweisen, erhalten wird.

9. Verfahren nach einem der Ansprüche 1 bis 8, wobei der Algorithmus oder die mathematische Funktion unter Verwendung eines maschinellen Lernansatzes erhalten wird.

10. Verfahren nach Anspruch 9, wobei der maschinelle Lernansatz die folgenden Schritte einbezieht:
(i) Eingeben der Level der Metaboliten in Zuckerrübenkeimlingen, für die der Zuckergehalt in den resultierenden voll ausgewachsenen Zuckerrüben bekannt ist, und
(ii) Berechnen eines Algorithmus oder einer mathematischen Funktion basierend auf den Leveln, der/die geeignet ist, den Zuckergehalt in der voll ausgewachsenen Zuckerrübe vorherzusagen.

11. Verfahren nach Anspruch 9, wobei der maschinelle Lernansatz die folgenden Schritte einbezieht:
(i) Eingeben der Level der Metaboliten in Zuckerrübenkeimlingen, die zu zwei oder mehr Kategorien von Zuckergehalt in den resultierenden voll ausgewachsenen Zuckerrüben gehören, und
(ii) Berechnen eines Algorithmus oder einer mathematischen Funktion basierend auf den Leveln, der/die geeignet ist, zwischen den zwei oder mehr Kategorien von Zuckergehalt in der voll ausgewachsenen Zuckerrübe zu unterscheiden.

12. Verfahren nach Anspruch 11, wobei die Kategorien von Zuckergehalt in den resultierenden voll ausgewachsenen Zuckerrüben aus der Gruppe ausgewählt sind, die aus einem bekannten niedrigen Zuckergehalt, einem bekannten mittleren Zuckergehalt und einem bekannten hohen Zuckergehalt besteht.

13. Verfahren nach Anspruch 12, wobei der maschinelle Lernansatz die folgenden Schritte einbezieht:
(i) Eingeben der Level der Metaboliten in Zuckerrübenkeimlingen, von denen bekannt ist, dass sie einen hohen Zuckergehalt in den resultierenden voll ausgewachsenen Zuckerrüben aufweisen, und der Level der Metaboliten in Zuckerrübenkeimlingen, von denen bekannt ist, dass sie einen niedrigen Zuckergehalt in den resultierenden voll ausgewachsenen Zuckerrüben aufweisen, und
(ii) Berechnen eines Algorithmus oder einer Funktion basierend auf den Leveln, der/die geeignet ist, zwischen der Kategorie eines hohen Zuckergehalts und der Kategorie eines niedrigen Zuckergehalts in der voll ausgewachsenen Zuckerrübe zu unterscheiden.

14. Verwendung mindestens zweier Metaboliten, die aus der Gruppe ausgewählt sind, die aus Var878, Var62, Var37 und Var19 besteht, zum Vorhersagen des Zuckergehalts in einer voll ausgewachsenen Zuckerrübe, wobei Var878, Var62, Var37 und Var19 wie in Anspruch 1 definiert sind.

## Revendications

1. Procédé de prédiction de la teneur en sucre d'une betterave à sucre arrivée à maturité comprenant les étapes de :
(i) détermination du taux de chacun d'au moins deux métabolites choisis dans le groupe constitué par Var878, Var62, Var37 et Var19 dans un échantillon obtenu à partir d'une plantule de betterave à sucre,
Var878 étant l'acide 2-oxo-glutarique, ayant une masse précise de 146,0218 Da ± 3 ppm, et un temps de rétention de 0,399 min ± 0,25 min en chromatographie liquide (CL),
Var62 étant l'acide phosphorique, ayant un indice de rétention de 1262,34 ± 2, et présentant des fragments ayant une masse nominale de 314, 299, 211, 283 et 225 Da en spectrométrie de masse (SM),
Var37 étant l'oxamide, ayant un indice de rétention de 1262,34 ± 2, et présentant des fragments ayant une masse nominale de 102, 189, 117, 217 et 131 Da en spectrométrie de masse (SM),
Var19 étant l'acide fumarique, ayant un indice de rétention de 1262,34 ± 2, et présentant des fragments ayant une masse nominale de 245, 115, 217, 143 et 133 Da en spectrométrie de masse (SM), et
(ii) la comparaison du taux de chaque métabolite à une référence, ladite comparaison permettant la prédiction de la teneur en sucre dans la betterave à sucre arrivée à maturité, et/ou l'application d'un algorithme ou d'une fonction mathématique aux taux des métabolites, ladite application permettant la prédiction de la teneur en sucre de la betterave à sucre arrivée à maturité.

2. Procédé selon la revendication 1, ledit taux de chacun des métabolites
(i) Var878 et Var62,
(ii) Var878 et Var37,
(iii) Var878 et Var19,
(iv) Var62 et Var37,
(v) Var62 et Var19,
(vi) Var37 et Var 19,
(vii) Var878, Var62 et Var37,
(viii) Var878, Var62 et Var19,
(ix) Var878, Var37 et Var19,
(x) Var62, Var37 et Var19, ou
(xi) Var878, Var62, Var37 et Var19
étant déterminé.

3. Procédé selon l'une quelconque des revendications 1 ou 2, ladite référence étant le taux dudit métabolite dans des plantules de betterave à sucre connues pour présenter
une teneur élevée en sucre,
une teneur moyenne en sucre, ou
une faible teneur en sucre
dans les betteraves à sucre arrivées à maturité résultantes.

4. Procédé selon l'une quelconque des revendications 1 ou 2, ladite référence étant le taux dudit métabolite dans des plantules de betterave à sucre pour lesquelles la teneur en sucre dans les betteraves à sucre arrivées à maturité résultantes est connue.

5. Procédé selon l'une quelconque des revendications 1 à 4, ladite fonction mathématique ou ledit algorithme étant obtenu à partir des taux desdits métabolites dans des plantules de betterave à sucre pour lesquelles la teneur en sucre dans les betteraves à sucre arrivées à maturité résultantes est connue.

6. Procédé selon l'une quelconque des revendications 1 à 4, ladite fonction mathématique ou ledit algorithme étant obtenu à partir des taux desdits métabolites dans des plantules de betterave à sucre appartenant à deux catégories ou plus de teneur en sucre dans les betteraves à sucre arrivées à maturité résultantes.

7. Procédé selon la revendication 6, lesdites catégories de teneur en sucre dans les betteraves à sucre arrivées à maturité résultantes étant choisies dans le groupe constitué par une faible teneur en sucre connue, une teneur moyenne en sucre connue et une teneur élevée en sucre connue.

8. Procédé selon la revendication 7, ladite fonction mathématique ou ledit algorithme étant obtenu à partir des taux desdits métabolites dans des plantules de betterave à sucre connues pour présenter une teneur élevée en sucre dans les betteraves à sucre arrivées à maturité résultantes, et
des taux desdits métabolites dans les plantules de betterave à sucre connues pour présenter une faible teneur en sucre dans les betteraves à sucre arrivées à maturité résultantes.

9. Procédé selon l'une quelconque des revendications 1 à 8, ladite fonction mathématique ou ledit algorithme étant obtenu à l'aide d'une méthode d'apprentissage machine.

10. Procédé selon la revendication 9, ladite méthode d'apprentissage machine impliquant les étapes suivantes :
(i) l'entrée des taux desdits métabolites dans des plantules de betterave à sucre pour lesquelles la teneur en sucre dans les betteraves à sucre arrivées à maturité résultantes est connue, et
(ii) le calcul d'une fonction mathématique ou d'un algorithme sur la base desdits taux qui sont appropriés pour prédire la teneur en sucre de la betterave à sucre arrivée à maturité.

11. Procédé selon la revendication 9, ladite méthode d'apprentissage machine impliquant les étapes suivantes :
(i) l'entrée des taux desdits métabolites dans des plantules de betterave à sucre appartenant à deux catégories ou plus de teneur en sucre dans les betteraves à sucre arrivées à maturité résultantes, et
(ii) le calcul d'une fonction mathématique ou d'un algorithme sur la base desdits taux qui sont appropriés pour faire la distinction entre deux catégories ou plus de teneur en sucre dans la betterave à sucre arrivée à maturité.

12. Procédé selon la revendication 11, lesdites catégories de teneur en sucre dans les betteraves à sucre arrivées à maturité résultantes étant choisies dans le groupe constitué par une faible teneur en sucre connue, une teneur moyenne en sucre connue et une teneur élevée en sucre connue.

13. Procédé selon la revendication 12, ladite méthode d'apprentissage machine impliquant les étapes suivantes :
(i) l'entrée des taux desdits métabolites dans des plantules de betterave à sucre connues pour présenter une teneur élevée en sucre dans les betteraves à sucre arrivées à maturité résultantes et des taux desdits métabolites dans des plantules de betterave à sucre connues pour présenter une faible teneur en sucre dans les betteraves à sucres arrivées à maturité résultantes, et
(ii) le calcul d'une fonction mathématique ou d'un algorithme sur la base desdits taux qui sont appropriés pour faire la distinction entre la catégorie de teneur élevée en sucre et la catégorie de faible teneur en sucre dans la betterave à sucre arrivée à maturité.

14. Utilisation d'au moins deux métabolites choisis dans le groupe constitué par Var878, Var62, Var37 et Var19 pour la prédiction de la teneur en sucre dans une betterave à sucre arrivée à maturité, Var878, Var62, Var37 et Var19 étant tels que définis selon la revendication 1.
